(19) Europäisches Patentamt
European Patent Office
Office européen des brevets

(11) Numéro de publication : 0 353 131 B1

# (12) FASCICULE DE BREVET EUROPEEN

(45) Date de publication du fascicule du brevet : **15.04.92 Bulletin 92/16**

(51) Int. Cl.[5] : **C07C 209/10,** C07C 211/48

(21) Numéro de dépôt : **89402075.9**

(22) Date de dépôt : **21.07.89**

(54) **Procédé de préparation de N. alkyl et N. allyl anilines.**

(30) Priorité : **29.07.88 FR 8810250**

(43) Date de publication de la demande : **31.01.90 Bulletin 90/05**

(45) Mention de la délivrance du brevet : **15.04.92 Bulletin 92/16**

(84) Etats contractants désignés : **AT BE CH DE ES FR GB GR IT LI LU NL SE**

(56) Documents cités :
**EP-A- 0 205 391**
**CH-A- 514 604**
**FR-A- 2 305 434**

(73) Titulaire : **RHONE POULENC CHIMIE**
**25 Quai Paul Doumer**
**F-92408 Courbevoie Cédex (FR)**

(72) Inventeur : **Desmurs, Jean**
**La Jonquière Route de Ternay**
**F-69360 Communay (FR)**
Inventeur : **Lecouve, Jean-Pierre**
**23e, rue de l'Oratoire**
**F-69300 Caluire (FR)**

(74) Mandataire : **Vignally, Noel et al**
**Rhône-Poulenc Chimie Service Brevets**
**Chimie 25 Quai Paul Doumer**
**F-92408 Courbevoie Cédex (FR)**

EP 0 353 131 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de N. alkyl et N. allyl anilines. Elle concerne plus particulièrement la préparation de N. allylanilines et encore plus préférentiellement la préparation de N. monoallylanilines.

La préparation des N monoallylanilines est particulièrement importante dans le cas de la trifluorométhylaniline car le dérivé monoallylique obtenu est un intermédiaire important dans la synthèse d'un herbicide tel que décrit dans le brevet FR 2 305 434. Selon ce brevet, pour préparer l'herbicide désiré, la N.metatrifluorométhylphényl-1 chloro-3 chlorométhyl-4 pyrrolidone-2, il est nécessaire de partir d'une trifluorométhylaniline dont un des atomes d'hydrogène est protégé par un groupe acétyl avant de procéder à l'allylation, ceci afin d'éviter la formation de produits secondaires de diallylation qui sont inutilisables.

L'industrie cherche depuis longtemps à accéder à la monoallyltrifluorométhylaniline directement en une seule étape, au lieu des trois étapes décrites dans le brevet FR 2 305 434, avec de bons rendements calculés sur la matière première engagée la metatrifluorométhylaniline qui est un composé très onéreux que l'industrie ne veut pas perdre.

Une première solution à ce problème a été proposée dans le brevet US 4 701 560 qui décrit un procédé d'allylation de la metatrifluorométhylaniline en milieu biphasique, eau-solvant organique en présence d'une base minérale choisie parmi les carbonates ou la soude et en présence de quantités catalytiques d'une amine tertiaire quaternisable. Pour obtenir une faible quantité de produits secondaires diallyliques, il est nécessaire de limiter le taux de transformation de la metatrifluorométhylaniline et donc de travailler en présence d'un défaut d'halogénure d'allyle, il est précisé dans ce texte que le rapport metatrifluorométhylaniline à l'halogénure d'allyle est de préférence d'environ 2. Les rendements en N monoallylaniline calculés sur la metatrifluorométhylaniline introduite ne dépasse pas 40 % ce qui est insuffisant pour une bonne rentabilité économique du procédé.

Les réactions d'allylation sur d'autres anilines que la metatrifluorométhylaniline sont décrites par exemple dans le brevet US 2 286 678 qui décrit notamment l'allylation de la parahydroxyaniline dans un milieu constitué d'un alcool et en présence de carbonate comme agent de neutralisation.

Les rendements annoncés en N monoallylhydroxyaniline ne dépassent pas ceux du brevet précédent et il y a en outre formation d'une quantité non négligeable de dérivés diallyliques que nous cherchons à éviter. Cette technique n'est donc pas transposable dans notre cas.

Il est également décrit dans le brevet US 3 668 254 un procédé qui consiste à allyler l'amino-4 diphénylamine avec le dichloro-2,3 propène en présence de triethylamine. Les rendements annoncés comme dans les deux cas précédents ne dépassent pas 40 %. En plus, la triethylamine est utilisée en quantité plus que stoechiométrique par rapport à l'halogénure d'allyle. Cette technique d'un point de vue économique est doublement peu intéressante, les rendements sont faibles et le coût des matières premières engagées est trop élevé.

Le brevet US 3 819 708 décrit l'alkylation de paraphénylènediamines dans divers solvants en présence d'une amine tertiaire telle que la triethylamine ou d'une base minérale comme agent de neutralisation de l'hydracide formé. Les agents alkylants qui sont décrits sont beaucoup moins réactifs que les halogénures d'allyle et le problème de dialkylation est donc beaucoup moins important. La sélectivité c'est-à-dire le rendement en produit monoalkylé par rapport aux dérivés dialkylés n'est jamais décrite.

Malgré l'existence d'une abondante littérature décrivant l'alkylation ou l'allylation de diverses anilines, aucun procédé n'a jamais permis de résoudre le problème évoqué dans la présente inventions c'est-à-dire avoir un bon taux de transformation de l'aniline de départ et obtenir une bonne sélectivité aniline monosubstituée sur l'azote par rapport à l'aniline disubstituée.

La présente invention a permis d'atteindre cet objectif.

Elle a pour objet un procédé de N monoalkylation et N. monoallylation d'une aniline caractérisée en ce que l'on met en contact l'aniline et un agent d'alkylation ou d'allylation dans un solvant organique en phase liquide homogène en présence d'une quantité catalytique d'un onium et d'une quantité stoechiométrique d'une base non quaternisable.

L'agent d'alkylation ou d'allylation est choisi parmi les halogénures ou les sulfates d'alkyle dont la chaîne alkyle peut comporter des insaturations, peut être linéaire ou ramifiée et peut en plus comporter des substituants choisis parmi les radicaux halogéno, aryle, aralkyle, halogénoaryle, nitroaryle.

On préfère parmi les halogénures utiliser les chlorures et les bromures et tout particulièrement les chlorures car ils sont les moins chers.

Parmi les halogénures d'alkyle et d'allyle la présente invention vise tout particulièrement les halogénures d'allyle car ce sont vis à vis de l'alkylation des agents peu réactifs surtout en ce qui concerne les chlorures.

On peut citer parmi les agents d'alkylation et d'allylation :

– le chlorure d'allyle

– le bromure d'allyle
– le chlorure de benzyle
– le bromure de benzyle
– le bromure d'isopropyle
– le chlorure de crotyle
– le chloro-1 butène-2

Le procédé de la présente invention s'applique à l'ensemble des anilines, il est cependant particulièrement intéressant pour les anilines peu basiques c'est-à-dire pour les ions anilinium présentant un pKa inférieur à 4,5.

Néanmoins le procédé de l'invention présente un avantage pour l'alkylation de toutes les anilines. Dans le cas d'anilines réactives dont la forme protonée présente un pKa supérieur à 4,5 l'utilisation d'onium permet de raccourcir considérablement le temps de réaction ou la température de réaction, ces deux critères étant très souvent liés l'un à l'autre.

Les anilines préférées c'est-à-dire présentant un pKa inférieur à 4,5 sont représentées par la formule (I) suivante :

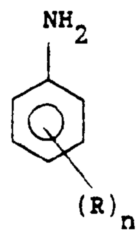

(I)

dans laquelle

– R représente :
  . un halogène
  . un groupe - A $C_n X_{2n+1}$ où X représente un halogène, A une liaison covalente, un atome d'oxygène ou de soufre,
  . un groupe nitro
– n est égal à 0,1 ou 2

On peut citer parmi les anilines de formule (I)

– l'aniline
– les chloroanilines
– les fluoroanilines
– les nitranilines
– les trihalogénométhylanilines
– les trihalogénométhoxyanilines
– les trihalogénométhylthioanilines

Les oniums utilisés dans le procédé de l'invention sont ceux dérivant notamment de l'azote, du phosphore, de l'arsenic, du soufre, du sélénium, de l'oxygène, du carbone ou de l'iode. Ces oniums sont coordinés à des restes hydrocarbonés. Les ions onium dérivant de l'azote, du phosphore ou de l'arsenic seront quadricoordinés, les ions onium dérivant du soufre, du sélénium, de l'oxygène, du carbone ou de S=0 seront tricoordinés.

Les restes hydrocarbonés coordinés à ces différents éléments sont des radicaux alkyles, alcényles, aryles, cycloalkyles, arylalkyles éventuellement substitués, deux restes hydrocarbonés coordinés pouvant former ensemble un radical unique divalent.

Parmi les oniums pouvant être utilisés dans le présent procédé, conviennent particulièrement ceux répondant à l'une des formules générales suivantes :

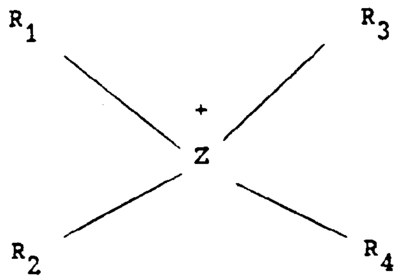

(II)

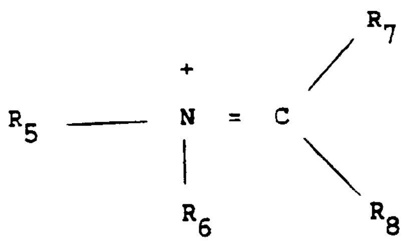

(III)

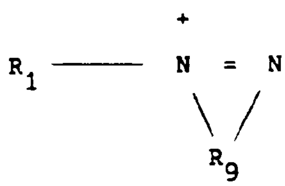

(III bis)

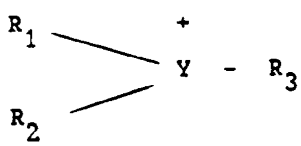

(IV)

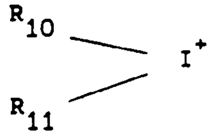

(V)

dans lesquelles :

- Z représente N, P ou As ;
- Y représente S, O, Se, S=0 ou C ;
- $R_1$, $R2_2$, $R_3$, et $R_4$, identiques ou différents représentent :
  . un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;

. un radical alcényle, linéaire ou ramifié, ayant 2 à 12 atomes de carbone ;

. un radical aryle ayant 6 à 10 atomes de carbone, éventuellement substitué par un ou plusieurs groupements ou atomes alkyles ayant 1 à 4 atomes de carbone, alkoxy, alkoxycarbonyle, le radical alkoxy ayant 1 à 4 atomes de carbone, ou halogène ;

. deux desdits radicaux $R_1$ à $R_4$ pouvant former ensemble un radical alkylène, acénylène ou alcadiènylène, linéaire ou ramifié ayant de 3 à 6 atomes de carbone ;

– $R_5$, $R_6$, $R_7$, $R_8$ sont identiques ou différents et représentent :

. un radical alkyle linéaire ou ramifié contenant de 1 à 4 atomes de carbone ;

. les radicaux $R_7$ et $R_8$ pouvant former ensemble un radical alkylène contenant de 3 à 6 atomes de carbone ;

. les radicaux $R_8$ et $R_7$ ou $R_8$ et $R_8$ pouvant former ensemble un radical alkylène, alcénylène ou alcadiènylène, contenant 4 atomes de carbone et constituant avec l'atome d'azote un hétérocycle azoté ;

– $R_9$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_1$.

– $R_{10}$ et $R_{11}$ représentent des radicaux aryles identiques ou différents

A titre d'exemples d'oniums répondant à la formule (II) on peut citer :

– tétraméthylammonium,
– triéthylméthylammonium,
– tributylméthylammonium,
– triméthylpropylammonium,
– tétraéthylammonium,
– tétrabutylammonium,
– dodécyltriméthylammonium,
– méthyltrioctylammonium,
– heptyltributylammonium,
– tétrapropylammonium,
– tétrapentylammonium,
– tétrahexylammonium,
– tétraheptylammonium,
– tétraoctylammonium,
– tétradécylammonium,
– butyltripropylammonium,
– méthyltributylammonium,
– pentyltributylammonium,
– méthyldiéthylpropylammonium,
– éthyldiméthylpropylammonium,
– tétradodécylammonium,
– tétraoctadécylammonium,
– hexadécyltriméthylammonium,
– benzyltriméthylammonium,
– benzyldiméthylpropylammonium,
– benzyldiméthyloctylammonium,
– benzyltributylammonium,
– benzyltriéthylammonium,
– phényltriméthylammonium,
– benzyldiméthyltétradécylammonium,
– benzyldiméthylhexadécylammonium,
– diméthyldiphénylammonium,
– méthyltriphénylammonium,
– butène-2-yltriéthylammonium,
– N, N-diméthyl-tétraméthylèneammonium,
– N, N-diéthyl-tétraméthylèneammonium,
– tétraméthylphosphonium,
– tétrabutylphosphonium,
– éthyltriméthylphosphonium,
– triméthylpentylphosphonium,
– octyltriméthylphosphonium,

– dodécyltriméthylphosphonium,
– triméthylphénylphosphonium,
– diéthyldiphénylphosphonium,
– dicyclohexyldiméthylphosphonium,
– diméthyldiphénylphosphonium,
– cyclohexyltriméthylphosphonium,
– triéthylméthylphosphonium,
– méthyltri(isopropyl)phosphonium,
– méthyltri(n-propyl)phosphonium,
– méthyltri(n-butyl)phosphonium,
– méthyltri(méthyl-2 propyl)phosphonium,
– méthyltricyclohexylphosphonium,
– méthyltriphénylphosphonium,
– méthyltribenzylphosphonium,
– méthyltri(méthyl-4 phényl)phosphonium,
– méthyltrixylylphosphonium,
– diéthylméthylphénylphosphonium,
– dibenzylméthylphénylphosphonium,
– éthyltriphénylphosphonium,
– tétraéthylphosphonium,
– éthyltri(n-propyl)phosphonium,
– triéthylpentylphosphonium,
– hexadécyltributylphosphonium,
– éthyltriphénylphosphonium,
– n-butyltri(n-propyl)phosphonium,
– butyltriphénylphosphonium,
– benzyltriphénylphosphonium,
– (B-phényléthyl)diméthylphénylphosphonium,
– tétraphénylphosphonium,
– triphényl(méthyl-4 phényl)phosphonium,
– tétrakis(hydroxyméthyl)phosphonium,
– tétraphénylarsonium.

Parmi les oniums répondant aux formules (III) et (III bis) on peut citer :

– N-méthylpyridinium,
– N-éthylpyridinium,
– N-hexadécylpyridinium,
– N-méthylpicolinium,
– triphényl-1,2,4 triazolium.

A titre d'exemple d'onium organiques répondant à la formule (IV), on peut citer les cations :

– triméthylsulfonium,
– triéthylsulfonium,
– triphénylsulfonium,
– triméthylsulfoxonium,
– triphénylcarbénium,
– triéthyloxonium.

A titre d'exemple d'onium répondant à la formule (V), on peut citer :

– diphényliodonium,
– diméthoxy-4,4′ diphényliodonium (ou les composés décrits dans JACS 1958, 81, 342),
– diphényliodonium 2-carboxylate. J6 3005040.

Dans la classe des oniums on préfère utiliser les oniums ayant un poids moléculaire compris entre 150 et 400 et de préférence compris entre 200 et 300. Parmi ces oniums conviennent tout particulièrement les ammoniums dont les quatre groupes alkyles sont semblables et possèdent quatre à cinq atomes de carbone.

L'onium peut être associé à un contre ion divers : on peut citer les halogénures, les hydroxyles, les hydrogénosulfates, les trifluorométhanesulfonate, les hexachloroantimonates.

L'onium peut être soluble dans le milieu réactionnel, on aura alors une réaction en milieu homogène ou insoluble sous forme solide, on aura alors une réaction en milieu biphasique solide-liquide.

L'onium peut aussi être supporté sur une résine minérale ou polymérique.

On peut citer parmi les oniums "supportés"

– le fluorure de tétrabutylammonium sur gel de silice
– le chlorure de tributylammonium sur polymère commercialisé par exemple par la société FLUKA
– le chlorure de méthyltributylphosphonium lié à du polystyrène commercialisé par exemple par la société FLUKA
– le bromure de benzyltrimethylammonium sur polymère

La réaction aura alors lieu en milieu biphasique solide-liquide.

La base utilisée dans le procédé de l'invention sert à neutraliser l'hydracide libéré au cours de l'alkylation ou l'allylation. Elle peut être choisie parmi les bases minérales telles que l'hydroxyde de sodium, les carbonates, ou parmi les bases organiques telles que l'acétate de sodium, ou les amines tertiaires non quaternisables.

On entend par amines tertiaires non quaternisables l'ensemble des amines tertiaires présentant au moins une chaîne alkyle ramifiée et de préférence au moins deux chaînes alkyles ramifiées. On peut citer parmi ces amines à titre d'exemple :

– la diisopropylallylamine
– la diisopropyléthylamine
– la triisopropylamine

On préfère parmi l'ensemble des bases citées utiliser la diisopropyléthylamine.

L'onium peut être apporté totalement synthétisé dans le milieu réactionnel ou être préparé "in situ". Dans ce dernier cas, on met en oeuvre une quantité catalytique d'une amine tertiaire quaternisable qui en présence de l'halogénure d'alkyle ou d'allyle donnera l'ammonium désiré. Cette amine est de préférence la triéthylamine.

Le solvant constitutif du milieu réactionnel doit solubiliser l'aniline et l'halogénure d'alkyle ou d'allyle, l'onium ou la base pouvant être solide ne sont pas toujours solubilisés par le milieu réactionnel, néanmoins il est préférable qu'ils soient solubles tous les deux dans le milieu réactionnel.

Les solvants sont choisis parmi :

– les hydrocarbures aliphatiques tels que
  . l'hexane
  . le cyclohexane
  . l'heptane
  . l'octane
– les hydrocarbures aromatiques tels que
  . le toluène
  . le xylène
– les hydrocarbures halogénés tels que
  . le chloroforme
  . le chlorure de méthylène
  . le chlorobenzène
  . le tétrachlorure de carbone
  . le dichloroéthane
– les alcools tels que
  . l'éthanol
  . l'isopropanol
  . le butanol
  . l'octanol
– les solvants aprotiques polaires tels que
  . le N,N diméthylformamide
  . l'acétonitrile
  . la N méthylpyrrolidone
– les amines tertiaires non quaternisables telles que
  . la diisopropylethylamine

On préfère parmi l'ensemble des solvants cités utiliser l'heptane ou la disopropyléthylamine.

Pour une meilleure mise en oeuvre de l'invention on préfère utiliser une quantité environ stoechiométrique d'halogénure d'alkyle ou d'allyle par rapport à l'aniline. Quand la base est utilisée pour former "in situ" l'onium la quantité d'halogénure d'alkyle ou d'allyle excédera la stoechiométrie par rapport à l'aniline.

L'onium est utilisé en quantité catalytique c'est-à-dire selon un rapport molaire compris entre 0,025 et 0,2 par rapport à l'aniline.

La température de réaction est avantageusement comprise entre 0° et 150°C et de préférence entre 25 et 80°C.

Elle variera en fonction des réactifs mis en présence surtout en fonction du pKa de l'anilinium et de la nature de l'halogénure.

La pression réactionnelle est de préférence la pression atmosphérique.

La durée de réaction varie entre une et quelques heures.

La présente invention sera plus complètement décrite à l'aide des exemples suivants qui ne doivent pas être considérés comme limitatifs de l'invention.

Dans les exemples suivants on entend par taux de transformation (TT).

$$TT = \frac{\text{nombre de moles d'aniline transformées}}{\text{nombre de moles d'aniline introduites}} \ \%$$

$$RT = \frac{\text{nombre de moles de produit formé}}{\text{nombre de moles d'aniline transformées}} \ \%$$

$$\text{Sélectivité} = \frac{\text{Quantité de N allyl ou N alkyl formé}}{\text{Quantité de N allyl ou de N alkyl formé + quantité de diallyl ou de dialkyl formé}}$$

EXEMPLES 1 A 12 ET COMPARATIFS 1 A 12

Dans un réacteur de 30 ml, on charge :

0,64 g de m-trifluorométhylaniline (4 mM)

0,3 g de chlorure d'allyle (4 mM)

2 ml de solvant

0,51 g de diisopropyléthylamine (4 mM)

et éventuellement du bromure d'allyltriéthylammonium (0,4 mM). Le mélange réactionnel est chauffé 3 h 30 à 80°. En fin de réaction, après refroidissement, on ajoute 5 ml de soude N. Les produits organiques sont extraits par 3 x 10 ml d'éther isopropylique.

La phase organique rassemblée est diluée à 50 ml dans une fiole jaugée pour être dosée en CPG.

| Essai | Onium | Solvant | $\varepsilon$ | TTmTFMA | RT N allyle | RT diallyle | Bilan |
|---|---|---|---|---|---|---|---|
| 1 | Oui | Heptane | 1,8 | 77 % | 82 % | 6,5 % | 96 % |
| C 1 | Non | | | 7,5 % | 100 % | 0 % | 100 % |
| 2 | Oui | $CCl_4$ | 2,2 | 25 % | 100 % | Traces | 100 % |
| C 2 | Non | | | 0 % | 0 % | 0 % | 100 % |
| 3 | Oui | Toluène | 2,4 | 67,5 % | 77 % | 4,2 % | 87,2 % |
| C 3 | Non | | | 0 % | 0 % | 0 % | 100 % |
| 4 | Oui | éther isopropylique | 3,5 | 12,5 % | 100 % | Traces | 100 % |
| C 4 | Non | | | 3,5 % | 71 % | 28 % | 100 % |

| Essai | Onium | Solvant | ε | TTmTFMA | RT Nallyle | RT diallyle | Bilan |
|---|---|---|---|---|---|---|---|
| 5 | Oui | $CHCl_3$ | 5,1 | 18 % | 100 % | Traces | 97,5 % |
| C 5 | Non | | | 0 % | 0 % | 0 % | 100 % |
| 6 | Oui | $ClCH_2CH_2Cl$ | 10,5 | 30 % | 94 % | 6 % | 100 % |
| C 6 | Non | | | 2,5 % | 75 % | 24 % | 99 % |
| 7 | Oui | Octanol-1 | | 47 % | 66,4 % | 33,6 % | 104 % |
| C 7 | Non | | | 18,4 % | 100 % | Traces | 95 % |
| 8 | Oui | Butanol-1 | 18 | 66 % | 95 % | 7,6 % | 106 % |
| C 8 | Non | | | 25 % | 40 % | Traces | 87,5 % |
| 9 | Oui | Ethanol | 24,3 | 40 % | 75 % | 3 % | 81 % |
| C 9 | Non | | | 15 % | 100 % | 0 % | 100 % |
| 10 | Oui | DMF | 36,7 | 58,7 % | 70 % | 5,1 % | 85,7 % |
| C 10 | Non | | | 11 % | 100 % | 0 % | 100 % |
| 11 | Oui | $CH_3CN$ | 37,5 | 12,5 % | 100 % | 0 % | 88 % |
| C 11 | Non | | | 2,5 % | 100 % | 0 % | 87 % |
| 12 | Oui | Diisopropyl-ethylamine | | 62,6 % | 91,12 % | 7,94 % | 99,3% |
| C 12 | Non | | | 5 % | 100 % | 0 % | 100 % |

EXEMPLES COMPARATIFS 13 ET 14

Allylation par les sels d'oniums

On opère comme dans l'exemple 1 en chargeant :
0,64 g de trifluorométhylaniline (4 mM)
2 ml d'heptane
4 mM de chlorure d'onium
Les mélanges réactionnels sont chauffés 5 heures à 75°.

| Essais | Conditions | Onium | TT mTFMA | RT Nallyle | RT diallyle | Bilan |
|---|---|---|---|---|---|---|
| C 13 | 75°C - 5 h | 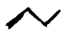 N $Et_3Cl$ | 0 | 0 | 0 | 100 |
| C 14 | 75°C - 5 h | 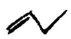 $PØ_3Cl$ | 0 | 0 | 0 | 100 |

Les halogénures d'onium ne sont pas allylant dans les conditions où l'on opère.

EXEMPLES 13 A 17

Influence de la quantité d'onium

On opère selon le mode opératoire général en chargeant :
0,64 g de m.trifluorométhylaniline (4 mM)
2 ml d'heptane
0,3 g de chlorure d'allyle (4 mM)
0,5 de diisopropyléthylamine (4 mM)
et des quantités de bromure d'allyltriéthylammonium allant de 0 à 4 mM. On laisse réagir durant 4 heures.

| Essai | 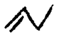 $\overset{+}{N}Et_3\overset{-}{Br}$ | TT mTFMA | RT Nallyle | RT diallyle | Bilan | Sélectivité |
|---|---|---|---|---|---|---|
| C 15 | | 7,5 % | 100 % | 0 % | 100 % | 100 % |
| 13 | 0,1 mM | 30 % | 90,2 % | 9,7 % | 100 % | 90,2 % |
| 14 | 0,4 mM | 59 % | 95,2 % | 4,8 % | 100 % | 95,2 % |
| 15 | 0,8 mM | 79 % | 92 % | 8 % | 102 % | 92 % |
| 16 | 1,6 mM | 70,4 % | 83,8 % | 6 % | 105 % | 94 % |
| 17 | 4 mM | 86,6 % | 87,1 % | 13 % | 100 % | 87,1 % |

EXEMPLES 18 A 30

Influence de la structure de l'ammonium

On opère selon le mode opératoire 1 en chargeant :

0,64 g de m.trifluorométhylaniline (4 mM)
2 ml d'heptane
0,3 g de chlorure d'allyle (4 mM)
0,5 g de diisopropyléthylamine (4 mM)

et 0,4 mM d'un ammonium. La réaction est effectuée à 80° pendant 4 heures.

| Essai | Onium | P.M | TTmTFMA | RTNallyle | RTdiallyle | Bilan | Sélectivité |
|---|---|---|---|---|---|---|---|
| 18 | $NH_4Cl$* | 53,5 | 10 % | 100 % | Traces | 100 % | 100 % |
| 19 | $NMe_4Cl$* | 109,6 | 17 % | 66 % | 2 % | 98 % | 97 % |
| 20 | (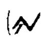)$_2NMe_2Cl$* | 161 | 47,5 % | 94,7 % | Traces | 100 % | 100 % |
| 21 | $Et_4NCl$ | 165,5 | 44,7 % | 96,7 % | 3,3 % | 106 % | 96,7 % |
| 22 | Ø$NMe_3Cl$ | 171,6 | 75 % | 83,3 % | 4 % | 92 % | 96,4 % |
| 23 | 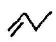 $NEt_3Cl$ | 177,5 | 58 % | 91,1 % | 8,9 % | 96 % | 91,1 % |
| 24 | Ø$CH_2NEt_3Cl$ | 227,8 | 62 % | 92 % | 4 % | 98 % | 96 % |
| 25 | $MeNBu_3Cl$ | 235,5 | 72,5 % | 81 % | 5,8 % | 90,5 % | 93,2 % |
| 26 | $Bu_4NCl$ | 277 | 70 % | 93 % | 5,3 % | 98,3 % | 94,5 % |
| 27 | Ø$CH_2NBu_3Cl$ | 312,5 | 72 % | 93 % | 7 % | 100 % | 93 % |
| 28 | 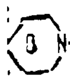$N-C_{16}H_{33}Cl$ | 358 | 57 % | 30 % | 0,8 % | 60 % | 97 % |
| 29 | $MeN(C_8H_{17})_3Cl$ | 404 | 42 % | 94 % | 1,7 % | 90 % | 97 % |
| 30 | Ø$CH_2NMe_2C_{16}H_{33}$ Cl | 414 | 40 % | 98 % | Traces | 100 % | 100 % |

* Ammoniums non solubles dans le milieu réactionnel.

## EXEMPLES 31 A 36

### Influence de la longueur de la chaîne de l'ammonium

On opère selon le mode opératoire 1 en chargeant :

0,64 g de m.trifluorométhylaniline (4mM)
2 ml d'heptane
0,3 g de chlorure d'allyle (4 mM)
0,5 g de diisopropyléthylamine (4 mM)

et 0,4 mM d'un chlorure d'ammonium. La réaction est effectuée à 80° pendant 4 heures.

| Essai | Onium | P.M | TTmTFMA | RTNallyle | RTdiallyle | Bilan | Sélectivité |
|---|---|---|---|---|---|---|---|
| 31 | $NH_4Cl$ | 53,5 | 10 % | 100 % | Traces | 100 % | 100 % |
| 32 | $NMe_4Cl$ | 109,6 | 17 % | 66 % | 2 % | 98 % | 97 % |
| 33 | $NEt_4Cl$ | 165,5 | 44,7 % | 96,7 % | 3,3 % | 106 % | 96,7 % |
| 34 | $NBu_4Cl$ | 277 | 70 % | 93 % | 5,3 % | 98,3 % | 94,5 % |
| 35 | $N(C_5H_{11})_4Cl$ | 333,5 | 60 % | 83,3 % | 4,1 % | 92,5 % | 95,3 % |
| 36 | $N(C_6H_{13})_4Cl$ | 389,5 | 52,5 % | 80 % | 4,7 % | 92 % | 94,5 % |

EXEMPLES 37 A 49

Influence du contre-ion de l'onium

On opère selon le mode opératoire 1 en chargeant :

0,64 g de m.trifluorométhylaniline (4 mM)
2 ml d'heptane
0,3 g de chlorure d'allyle (4 mM)
0,5 g de diisopropyléthylamine (4 mM)
et 0,4 mM d'un sel d'onium. La réaction est effectuée à 80° pendant 4 heures.

Cation : 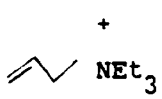

| Essai | Contre-ion | TT mTFMA | RT Nallyle | RT diallyle | Bilan | Sélectivité |
|---|---|---|---|---|---|---|
| 37 | $Cl^-$ | 58,3 % | 91,1 % | 8,9 % | 100 % | 91,1 % |
| 38 | $Br^-$ | 70 % | 78,6 % | 3,5 % | 87,5 % | 95,7 % |

Cation : 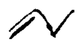 $PØ_3^+$

| Essai | Contre-ion | TT mTFMA | RT Nallyle | RT diallyle | Bilan | Sélectivité |
|---|---|---|---|---|---|---|
| 39 | $Cl^-$ | 53 % | 98 % | 2 % | 100 % | 98 % |
| 40 | $Br^-$ | 75 % | 93,3 % | 6,6 % | 100 % | 93,3 % |

Cation : $Et_4N^+$

| Essai | Contre-ion | TT mTPMA | RT Nallyle | RT diallyle | Bilan | Sélectivité |
|---|---|---|---|---|---|---|
| 41 | $Cl^-$ | 44 % | 96,7 % | 3,3 % | 106 % | 85,6 % |
| 42 | $Br^-$ | 77,5 % | 93,5 % | 6,4 % | 100 % | 93,5 % |

Cation : $Bu_4N^+$

| Essai | Contre-ion | TT mTPMA | RT Nallyle | RT diallyle | Bilan | Sélectivité |
|---|---|---|---|---|---|---|
| 43 | $OH^-$ | 42 % | 98 % | Traces | 100 % | 100 % |
| 44 | $ClO_4^-$ | 45 % | 99 % | Traces | 100 % | 100 % |
| 45 | $HSO_4^-$ | 54 % | 95,2 % | 4,8 % | 100,5 % | 95,2 % |
| 46 | $CF_3SO_3^-$ | 46 % | 88,8 % | 3,3 % | 96 % | 96,3 % |
| 47 | $Br_3^-$ | 68,5 % | 92 % | 8 % | 100 % | 92 % |
| 48 | $Cl^-$ | 70 % | 93 % | 5,3 % | 100 % | 94,5 % |
| 49 | $Br^-$ | 67,9 % | 93 % | 7 % | 100 % | 93 % |

EXEMPLES 50 A 54 ET COMPARATIF 50

Influence de la nature de l'onium ou de radicaux cations

On opère comme dans l'exemple 1 en chargeant :
0,64 g m.trifluorométhylaniline (4 mM)
0,3 g de chlorure d'allyle (4 mM)
2 ml d'heptane
0,51 g de diisopropyléthylamine (4 mM)
et 0,4 mM d'un onium. La réaction est effectuée à 80° en 4 heures.

| Essai | Onium | TT m TFMA | RT N.Allyle | RT,N,N.Diallyle |
|---|---|---|---|---|
| 50 | $(Ø)_3CSbCl_5$ | 43,6 % | 85,7 % | 9,3 % |
| 51 | $(Ø)_4PCl$ | 50 % | 95,8 % | 4,1 % |
| 52 | $(Ø)_2ICl$ | 43,6 % | 80 % | 3,5 % |
| 53 | $(Ø)_4AsCl$ | 61,4 % | 91,6 % | 5,4 % |
| 54 | $(BrØ)_3N^+ SbCl_6^-$ | 53,7 % | 78,4 % | 4,7 % |

EXEMPLES 55 A 58

Influence d'oniums supportés

On opère comme dans l'exemple 1 en chargeant :
0,64 g de m.trifluorométhylaniline (4 mM)
0,3 g de chlorure d'allyle (4 mM)
2 ml d'heptane
0,51 g de diisopropyléthylamine (4 mM)
et une quantité d'onium supporté. La réaction est effectuée à 80° en 4 heures.

| Essai | Onium | TT mTFMA | RT Nallyle | RT diallyle | Bilan | Sélectivité |
|---|---|---|---|---|---|---|
| 55 | Ⓟ $-CH_2-NBu_3Cl$ 0,45 mM de Cl | 57,7 % | 87,5 % | 6 % | 96 % | 90,5 % |
| 56 | Ⓟ $-CH_2-PBu_3Cl$ 0,39 mM de Cl | 72,6 % | 92,4 % | 6 % | 99 % | 93 % |
| 57 | Ⓟ $-C_6H_4CH_2N(CH_3)_3Br$ 0,67 mM de Br | 82 % | 79 % | 7,8 % | 89 % | 91 % |
| 58 | $Bu_4NF$ sur gel de silice 0,83 mM de F | 77 % | 80,5 % | 7,6 % | 90,7 % | 91,3 % |

Ⓟ est un polymère.

EXEMPLES 59 A 65

Influence de la base

On opère comme dans l'exemple 1 en chargeant :

0,64 g de m.trifluorométhylamine (4 mM)

0,6 g de chlorure d'allyle (8 mM)

2 ml d'heptane

4 mM d'une base

et eventuellement 0,4 ou 4 mM de bromure d'allyltriéthylammonium. La réaction est effectuée à 75° pendant 3 heures.

| Essai | Onium | Base | TT n.TFMA | RT N.Allyl | RT N,N.Diallyle |
|---|---|---|---|---|---|
| C 59 | Non | 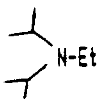 | 4,4 % | 100 % | |
| 59 | Oui (4 mM) | 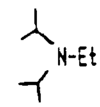 | 97 % | 54 % | 4,0 |
| C 60 | Non | AcONa | 4,8 | 0 | 0 |
| 60 | Oui (0,4 mM) | AcONa | 66,9 | 75,4 | 1,2 |
| 61 | Oui (4 mM) | AcONa | 96,7 | 49,9 | 50,2 |

Une deuxième série d'essais a été effectuée à 75° en 2 h 30 en chargeant :
0,64 g de m.trifluorométhylaniline
0,3 g de chlorure d'allyle (4 mM)
2 ml d'heptane
5 mM de base
0,4 mM de bromure d'allyltriéthylammonium

| Essai | Solvant | Temp/temps | Base | TT n.TFMA | RT N-Allyl | RT N,N.diallyl |
|---|---|---|---|---|---|---|
| 62 | Heptane | 75° - 2h30 | NaOH | 77,6 | 77,8 | 10 |
| 63 | Heptane | 75° - 2h30 | $K_2CO_3$ | 49,6 | 81,6 | 6,4 |
| 64 | Heptane | 75° - 2h30 | $Na_2CO_3$ | 50,5 | 76,6 | 6,2 |
| 65 | Heptane | 75° - 2h30 | 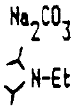 | 88,3 | 34,6 | 6,2 |

EXEMPLE 66

Essai de recyclage de l'ammonium supporté

1ère opération

Dans un réacteur, on charge
4 mM de m.TFMA
4 mM de chlorure d'allyle

2 ml de diisopropyléthylamine
et une quantité de

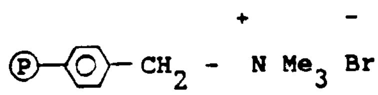

correspondant à 0,4 mM de $Br^-$

La réaction est effectuée à 80° pendant 4 heures. En fin de réaction, la résine est éliminée par filtration. Le filtrat est traité comme dans l'exemple 1.

Résultats

| | |
|---|---|
| TT mTFMA = | 87,5 % |
| RT Nallyle = | 48,5 % |
| RT diallyle = | 4 % |
| onium = | 58,7 % |
| Sélectivité = | 92 % |

2ème opération

On charge la résine filtrée précédemment avec :
4 mM de m.TFMA
4 mM de chlorure d'allyle
2 ml de diisopropyléthylamine.

La réaction, effectuée à 80° pendant 4 heures, est traitée comme précédemment.

Résultats

| | |
|---|---|
| TT mTFMA = | 80 % |
| RT Nallyle = | 73,1 % |
| RT diallyle = | 6,2 % |
| bilan = | 87,5 % |
| Séléctivité = | 92 % |

EXEMPLES 67 A 69

Influence du temps de réaction

On opère comme dans l'exemple 1 en chargeant :
0,64 g de m.trifluorométhylaniline (4 mM)
0,3 g de chlorure d'allyle (4 mM)
2 ml d'heptane
0,51 g de diisoprorpyléthylamine (4 mM)
0,4 mM de bromure de tetraéthylammonium.

La réaction est effectuée à 80° pendant des temps variables.

| Essai | Temps en mn | T.T m.TFMA | R.T Nallyle | R.T Diallyle | Bilan | Sélectivité |
|---|---|---|---|---|---|---|
| 67 | 240 | 77 % | 84,8 % | 9 % | 94,8 % | 90,3 % |
| 68 | 140 | 65 % | 89 % | 6,3 % | 96,6 % | 93,4 % |
| 69 | 60 | 48 % | 86 % | 3,6 % | 95,7 % | 96,5 % |

EXEMPLES 70 A 77

Influence de l'agent alkylant

a) Halogénure d'allyle

On opère comme dans l'exemple 1 en chargeant :

0,64 g de m.trifluorométhylaniline (4 mM)
4 mM d'un halogénure d'allyle
2 ml d'heptane
0,51 g de diisopropyléthylamine (4 mM)
et éventuellement 0,4 mM de bromure d'allyltriéthylammonium.

La réaction est poursuivie pendant 4 heures à 80°C.

| Essai | X 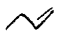 | 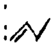 $NEt_3Br$ | T.T mTFMA | R.T Nallyle | R.T diallyle | Sélectivité |
|---|---|---|---|---|---|---|
| 70 | Cl 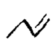 | Oui | 71,4 % | 82 % | 6 % | 93,2 % |
| 71 | Br 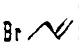 | Oui | 83 % | 83,3 % | 10,8 % | 85,2 % |

En l'absence d'onium, le taux de transformation est de 7,5 % pour le chlorure d'allyle et de 81,2 % pour le bromure d'allyle.

Pendant 2 h 30 à 25°C

| Essai | X 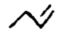 | 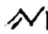 $NEt_3Br$ | T.T mTFMA | R.T Nallyle | R.T diallyle | Sélectivité |
|---|---|---|---|---|---|---|
| 72 | Br 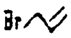 | Oui | 85,7 % | 76,6 % | 11,6 % | 86,8 % |

En l'absence d'onium, le taux de transformation est de 80 %.

Pendant 1 h à 25°C

| Essai | X 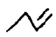 | 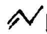 $NEt_3Br$ | T.T mTFMA | R.T Nallyle | R.T diallyle | Sélectivité |
|---|---|---|---|---|---|---|
| 73 | Br 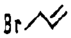 | Oui | 63 % | 84 % | 7 % | 92 % |

En l'absence d'onium, le taux de transformation est de 20 %.

b) Halogénure de benzyle

On opère comme dans l'exemple 1 en chargeant :

0,64 g de m.trifluorométhylaniline (4 mM)
4 mM d'un halogénure de benzyle
2 ml d'heptane
0,51 g de diisopropyléthylamine (4 mM)
et eventuellement 0,4 mM d'un bromure d'allyltriethylammonium.

On laisse la réaction se poursuivre à 80°C pendant 4 heures.

| Essai | $C_6H_5CH_2X$ | 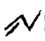 $\overset{+}{N}Et_3\overset{-}{Br}$ | T.T mTFMA | R.T.N benzyle | R.T dibenzyle | Sélectivité |
|---|---|---|---|---|---|---|
| 74 | $C_6H_5CH_2Cl$ | Oui | 95 % | 85 % | 15 % | 85 % |
| 75 | $C_6H_5CH_2Br$ | Oui | 85 % | 75 % | 25 % | 75 % |

En l'absence d'onium, le taux de transformation est de 85 % pour le chlorure de benzyle et de 90 % pour le bromure de benzyle.

A 25°C pendant 4 heures

| Essai | 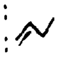 $\overset{+}{N}Et_3\overset{-}{Br}$ | T.T mTFMA | R.T. N benzyle | R.T dibenzyle | Sélectivité |
|---|---|---|---|---|---|
| 76 | Oui | 43,5 % | 100 % | 0 % | 100 % |

En l'absence d'onium, le taux de transformation est de 1 %.

c) Bromure d'isopropyle

On opère comme dans l'exemple 1 en chargeant :
0,64 g de m.trifluorométhylaniline (4 mM)
4 mM de bromure d'isopropyle (4 mM)
2 ml d'heptane
0,51 g de diisopropyléthylamine (4 mM)
et éventuellement 0,4 mM de bromure de d'allyltriéthylammonium. La réaction est effectuée à 80° pendant 4 heures.

| Essai | 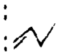 $\overset{+}{N}Et_3\overset{-}{Br}$ | T.T mTFMA | R.T.N isopropyle | R.T disopropyle | Sélectivité |
|---|---|---|---|---|---|
| 77 | Oui | 25,7 % | 100 % | 0 % | 100 % |

En l'absence d'onium, il n'y a aucune réaction.

EXEMPLES 78 A 84

Influence de l'amine

On opère comme dans l'exemple 1 en chargeant :
4 mM d'une amine
4 mM d'un chlorure d'allyle
2 ml d'heptane
4 mM de diisopropyléthylamine
0,4 mM de bromure d'allyltriéthylammonium
La réaction est effectuée à 80°C pendant 4 heures.

| Essai | X aniline | pKa | 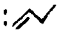 $N^+Et_3Br^-$ | TT X.aniline | RT Nallyle | RT diallyle |
|---|---|---|---|---|---|---|
| C 78 | 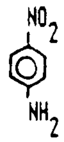 | 1 | Non | 0 % | | |
| 78 | | | Oui | 43,2 % | 100 % | 0 % |
| C 79 | 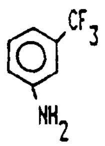 | 3,8 | Non | 7,5 % | 100 % | 0 % |
| 79 | | | Oui | 77 % | 82 % | 6,5 % |
| C 80 | 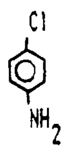 | 4,15 | Non | 68,5 % | 95 % | 5 % |
| 80 | | | Oui | 85 % | 81 % | 17 % |
| C 81 | 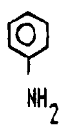 | 4,63 | Non | 90,3 % | 81,2 % | 19,8 % |
| 81 | | | Oui | 92,4 % | 75,3 % | 24,7 % |
| C 82 | 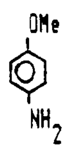 | 5,34 | Non | 87 % | 70 % | 30 % |
| 82 | | | Oui | 90 % | 60 % | 40 % |

La réaction a également été effectuée pendant des durées plus courtes.

| Essai | aniline | Temps | $N^+Et_3Br^-$ | TT aniline | RT Nallyle | RT diallyle |
|---|---|---|---|---|---|---|
| C 83 | 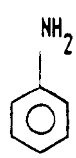 | 1 h | Non | 7,5 % | 100 % | 0 % |
| 83 | | 1 h | Oui | 67 % | 95 % | 5 % |
| C 84 | pKa = 4,63 | 4 h | Non | 90,3 % | 81,2 % | 19,8 % |
| 84 | | 4 h | Oui | 92,4 % | 75,3 % | 24,7 % |

## Revendications

1. Procédé de mono N alkylation ou allylation d'une aniline caractérisé en ce que l'on met en contact l'aniline et un agent d'alkylation ou d'allylation dans un solvant organique en phase liquide homogène en présence d'une quantité catalytique d'un onium et d'une quantité stoechiométrique d'une base non quaternisable.

2. Procédé selon la revendication 1 caractérisé en ce que l'agent d'alkylation ou d'allylation est choisi parmi les halogénures allyliques.

3. Procédé selon la revendication 2 caractérisé en ce que l'agent d'allylation est le chlorure d'allyle.

4. Procédé selon la revendication 1 caractérisé en ce que l'aniline présente un pKa inférieur à 4,5.

5. Procédé selon la revendication 4 caractérisé en ce que l'aniline est choisie parmi les halogénoanilines, les perhalogénoalkylanilines, les perhalogénoalcoxyanilines, les perhalogénoalkylthioanilines, les nitroanilines de formule (I).

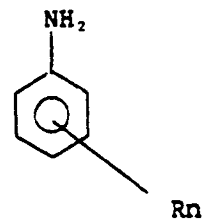

(I)

dans laquelle

– R représente
  . un halogène,
  . un groupe $-AC_nX_{2n+1}$ dans lequel X représente un halogène et A une liaison covalente, un atome d'oxygène ou de soufre,
  . un groupe $NO_2$,
– n est égal à 0,1 ou 2.

6. Procédé selon la revendication 1 caractérisé en ce que l'onium est choisi parmi les ammoniums, les phosphoniums, les sulfoxoniums, les sulfoniums, les carboniums, les arséniums, les oxoniums.

7. Procédé selon la revendication 6 caractérisé en ce que l'onium est choisi parmi les oniums ayant un poids moléculaire compris entre 150 et 400 et de préférence entre 200 et 300.

8. Procédé selon les revendications 6 et 7 caractérisé en ce que l'onium est un onium supporté.

9. Procédé selon la revendication 1 caractérisé en ce que la base non quaternisable est une base minérale ou organique.

10. Procédé selon la revendication 9 caractérisé en ce que la base non quaternisable est une base organique choisie parmi l'acétate de sodium, ou une amine tertiaire portant au moins un groupe alkyle ramifié et de préférence au moins deux groupes alkyles ramifiés.

11. Procédé selon la revendication 10 caractérisé en ce que la base est choisie parmi la diisopropylallylamine, la diisopropyléthylamine ou la triisopropylamine.

12. Procédé selon la revendication 11 caractérisé en ce que la base est la diisopropyléthylamine.

13. Procédé selon la revendication 1 caractérisé en ce que l'onium peut être formé au sein du milieu réactionnel par quaternisation d'une amine tertiaire.

14. Procédé selon la revendication 1 caractérisé en ce que le rapport molaire de l'agent alkylant ou allylant aniline est d'environ 1.

15. Procédé selon la revendication 1 caractérisé en ce que le rapport molaire de l'onium à l'aniline est comprise entre 0,025 et 0,20.

16. Procédé selon la revendication 1 caractérisé en ce que le solvant est choisi parmi les solvants hydrocarbonés aromatiques, aliphatiques, les alcools, les solvants aprotiques polaires, les amines tertiaires non quaternisables.

17. Procédé selon la revendication 16 caractérisé en ce que le solvant choisi est l'heptane ou la diisopropyléthylamine.

## Claims

1. Process for the N-monoalkylation or monoallylation of an aniline, characterized in that contacting takes place between aniline and an alkylating or allylating agent in an organic solvent in the homogeneous liquid phase in the presence of a catalytic quantity of an onium and a stoichiometric quantity of a non-quaternizable base.

2. Process according to claim 1, characterized in that the alkylating or allylating agent is chosen from among the allyl halides.

3. Process according to claim 2, characterized in that the allylating agent is allyl chloride.

4. Process according to claim 1, characterized in that the aniline has a pKa below 4.5.

5. Process according to claim 4, characterized in that the aniline is chosen from among haloanilines, perhaloalkylanilines, perhaloalkoxyanilines, perhaloalkylthioanilines and nitroanilines of formula (I)

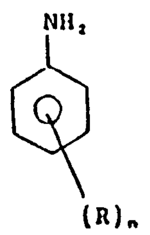

(I)

in which

- R represents
  - . a halogen
  - . a $-AC_nX_{2n+1}$ group, in which X represents a halogen and A a covalent bond, an oxygen or sulphur atom,
  - . a $NO_2$ group,
  - . n is equal to 0, 1 or 2.

6. Process according to claim 1, characterized in that the onium is chosen from among ammoniums, phosphoniums, sulphoxoniums, sulphoniums, carboniums, arseniums and oxoniums.

7. Process according to claim 6, characterized in that the onium is chosen from among oniums having a molecular weight between 150 and 400 and preferably between 200 and 300.

8. Process according to claims 6 and 7, characterized in that the onium is a supported onium.

9. Process according to claim 1, characterized in that the non- quaternizable base is a mineral or organic base.

10. Process according to claim 9, characterized in that the non- quaternizable base is an organic base chosen from among sodium acetate, or a tertiary amine carrying at least one branched alkyl group or preferably at least two branched alkyl groups.

11. Process according to claim 10, characterized in that the base is chosen from among diisopropylallylamine, diisopropylethylamine or triisopropylamine.

12. Process according to claim 11, characterized in that the base is diisopropylethylamine.

13. Process according to claim 1, characterized in that the onium can be formed within the reaction medium by the quaternization of a tertiary amine.

14. Process according to claim 1, characterized in that the molar ratio of the alkylating or allylating agent to the aniline is approximately 1.

15. Process according to claim 1, characterized in that the molar ratio of the onium to the aniline is between 0.025 and 0.20.

16. Process according to claim 1, characterized in that the solvent is chosen from among aromatic, aliphatic hydrocarbon solvents, alcohols, aprotic polar solvents and non-quaternizable tertiary amines.

17. Process according to claim 16, characterized in that the solvent is chosen from among heptane or diisopropylethylamine.

## Patentansprüche

1. Verfahren der N-Monoalkylierung oder N-Monoallylierung eines Anilins, dadurch gekennzeiichnet, daß man das Anilin und ein Alkylierungs- oder Allylierungsmittel in einem organischen Lösungsmittel in homogener flüssiger Phase in Gegenwart einer katalytischen Menge eines Oniums und einer stöchiometrischen Menge

einer nicht quaternisierbaren Base zusammenbringt.

2. Verfahren nach Anspruch 1, dadurch gekennzeichnet, daß das Alkylierungs- oder Allylierungsmittel unter den Allylhalogeniden ausgewählt wird.

3. Verfahren nach Anspruch 2, dadurch gekennzeichnet, daß das Allylierungsmittel Allylchlorid ist.

4. Verfahren nach Anspruch 1, dadurch gekennzeichnet, daß das Anilin einen pKa unter 4,5 aufweist.

5. Verfahren nach Anspruch 4, dadurch gekennzeichnet, daß das Anilin ausgewählt wird aus den Halogenanilinen, Perhalogenalkylanilinen, Perhalogenalkoxyanilinen, Perhalogenalkylthioanilinen und Nitroanilinen der Formel (I)

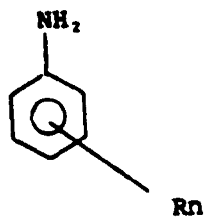

(I)

in der

– R
  . ein Halogen,
  . eine Gruppe $-AC_nX_{2n+1}$, in der X ein Halogen und A eine kovalente Bindung bedeuten,
  . ein Sauerstoff- oder Schwefelatom,
  . eine $NO_2$-Gruppe bedeutet und
– n gleich 0,1 oder 2 ist.

6. Verfahren nach Anspruch 1, dadurch gekennzeichnet, daß das Onium aus den Ammonium-, Phosphonium-, Sulfoxonium-, Sulfonium-, Carbonium-, Arsenium- und Oxoniumverbindungen ausgewählt wird.

7. Verfahren nach Anspruch 6, dadurch gekennzeichnet, daß das Onium aus den Oniumverbindungen mit einem Molekulargewicht im Bereich von 150 bis 400, vorzugsweise von 200 bis 300, ausgewählt wird.

8. Verfahren nach den Ansprüchen 6 und 7, dadurch gekennzeichnet, daß das Onium ein Träger-Onium ist.

9. Verfahren nach Anspruch 1, dadurch gekennzeichnet, daß die nicht quaternisierbare Base eine mineralische oder organische Base ist.

10. Verfahren nach Anspruch 9, dadurch gekennzeichnet, daß die nicht quaternisierbare Base eine organische Base ist, ausgewählt aus Natriumacetat oder einem tertiären Amin, das mindestens eine verzweigte Alkylgruppe, vorzugsweise mindestens zwei verzweigte Alkylgruppen, aufweist.

11. Verfahren nach Anspruch 10, dadurch gekennzeichnet, daß die Base aus Diisopropylallylamin, Diisopropylethylamin und Triisopropylamin ausgewählt wird.

12. Verfahren nach Anspruch 11, dadurch gekennzeichnet, daß die Base Diisopropylethylamin ist.

13. Verfahren nach Anspruch 1, dadurch gekennzeichnet, daß das Onium im Reaktionsmedium durch Quaternisierung eines tertiären Amins gebildet werden kann.

14. Verfahren nach Anspruch 1, dadurch gekennzeichnet, daß das Molverhältnis von Alkylierungs- oder Allylierungsmittel zu Anilin etwa 1 beträgt.

15. Verfahren nach Anspruch 1, dadurch gekennzeichnet, daß das Molverhältnis von Onium zu Anilin im Bereich von 0,025 bis 0,20 liegt.

16. Verfahren nach Anspruch 1, dadurch gekennzeichnet, daß das Lösungsmittel aus den aromatischen und aliphatischen Kohlenwasserstoffen, Alkoholen, aprotischen polaren Lösungsmitteln und nicht quaternisierbaren tertiären Aminen ausgewählt wird.

17. Verfahren nach Anspruch 16, dadurch gekennzeichnet, daß das ausgewählte Lösungsmittel Heptan oder Diisopropylethylamin ist.